# EUROPÄISCHE PATENTSCHRIFT

(11) **EP 1 421 120 B1**
(45) Veröffentlichungstag und Bekanntmachung des Hinweises auf die Patenterteilung: **25.04.2007**
(21) Anmeldenummer: 02758435.8
(22) Anmeldetag: 06.08.2002
(51) Int. Cl.: C08B 35/00, C08B 30/20, A61K 47/36, A61K 31/718, A01N 1/02, G01N 33/49, A61M 1/36

(54) **HYPERVERZWEIGTES AMYLOPEKTIN ZUM EINSATZ IN VERFAHREN ZUR CHIRURGISCHEN ODER THERAPEUTISCHEN BEHANDLUNG VON SÄUGERN ODER IN DIAGNOSTIZIERVERFAHREN, INSBESONDERE ZUR VERWENDUNG ALS PLASMAVOLUMENEXPANDER**
HYPERBRANCHED AMYLOPECTIN FOR USE IN METHODS FOR SURGICAL OR THERAPEUTIC TREATMENT OF MAMMALS OR IN DIAGNOSTIC METHODS, ESPECIALLY FOR USE AS A PLASMA VOLUME EXPANDER
AMYLOPECTINE HYPERRAMIFIEE DESTINEE A ETRE UTILISEE POUR LE TRAITEMENT CHIRURGICAL OU THERAPEUTIQUE DE MAMMIFERES OU DANS DES PROCEDES DE DIAGNOSTIC, ET EN PARTICULIER EN TANT QUE SUCCEDANE DE PLASMA

(30) Priorität: 22.08.2001 DE 10141099
(43) Veröffentlichungstag der Anmeldung: 26.05.2004
(73) Patentinhaber: Supramol Parenteral Colloids GmbH, 61191 Rosbach-Rodheim (DE)
(72) Erfinder: SOMMERMEYER, Klaus, 61191 Rosbach v.d.H. (DE)
(74) Vertreter: Luderschmidt, Schüler & Partner
(86) Internationale Anmeldenummer: PCT/EP2002/008757
(87) Internationale Veröffentlichungsnummer: WO 2003/018639

(56) Entgegenhaltungen:
- EP-A- 1 075 839
- WO-A-00/18893
- DE-A- 3 313 600
- GB-A- 1 279 356
- DATABASE CHEMABS [Online] CHEMICAL ABSTRACTS SERVICE, COLUMBUS, OHIO, US; "Manufacture of highly-branched starch inhibition of retrogradation of starch, and food containing the starch" retrieved from STN Database accession no. 135:317549 XP002222974 & JP 2001 294601 A (AKITA PREFECTURE) 23. Oktober 2001 (2001-10-23)
- GUNJA ZEENAT ET AL.: "Enzymic conversion of amyopectin into a glycogen-type polysaccharide" CHEM. & IND. , 1959, Seite 1017 XP008011341 London

## Beschreibung

Die vorliegende Erfindung betrifft den Einsatz von hyperverzweigtem Amylopektin.

Speziell richtet sich die Erfindung auf eine neuartige Verwendung von hyperverzweigtem Amylopektin, das einen bestimmten Verzweigungsgrad und ein bestimmtes Molekulargewicht Mw aufweist.

In der Geschichte der Entwicklung der Plasmavolumenexpander war es immer ein Ziel, die globuläre Struktur des natürlichen Trägers des kolloidosmotischen Druckes im Serum, des Albumins, zu erreichen. Dieser globulären Struktur kommt das Glycogen, welches ebenfalls als natürliches Speicherpolysaccharid im menschlichen Organismus vorkommt, nahe. Seine globuläre Struktur erreicht das Glycogen durch seinen sehr hohen Verzweigungsgrad. Strukturell stellt das Glycogen ein Glucosepolysaccharid dar mit in linearen Abschnitten α-1,4 glycosidischen Bindungen an denen α-1,6 glycosidische Verzweigungspunkte fixiert sind. Weil Glycogen selbst nicht als eine billige Rohstoffquelle zur Verfügung steht, schlug Wiedersheim 1957 vor, an dessen Stelle das geringer verzweigte Amylopektin als Ausgangsmaterial zur Herstellung des Plasmaexpanders Hydroxyethylstärke (HES) einzusetzen. Mittlerweile wird Hydroxyethylstärke in mehreren verschiedenen Typen sehr breit als Plasmaexpander eingesetzt. Die Entwicklung hat zu neuen Hydroxyethylstärke-Typen (HES-Typen) geführt, die einen optimalen Volumeneffekt aufweisen bei sonst minimalen Nebenwirkungen wie z. B. Beeinflussung der Gerinnung oder aber auch intermediäre Speicherung im Gewebe.

Die verschiedenen im Markt befindlichen HES-Typen unterschieden sich im Bezug auf Molekulargewicht, mittleren Substitutionsgrad und Substitutionsmuster.
Trotz des beachtlichen Fortschritts, der mit diesen Entwicklungen erreicht wurde, verbleiben einige Nachteile auch bei den in den letzten Jahren optimierten HES-Typen, vor allem die nicht vollständige Metabolisierbarkeit.

Es ist bekannt, dass die Hydroxyethyl-Ethergruppe chemisch aber auch metabolisch außerordentlich stabil ist, so dass diejenigen Anhydroglucose-Einheiten der Hydroxyethylstärke, die Hydroxyethyl-Ethergruppen tragen, praktisch nicht metabolisierbar sind. Weiterhin ist bekannt, dass nur diejenigen α-1,4 glycosidischen Bindungen im Hydroxyethyl-Stärkemolekül durch die Serum-α-Amylase gespalten werden können, die durch nicht substituierte Glucoseeinheiten gebildet werden. Aus diesem Grunde ist festzustellen, dass selbst bei den optimierten HES-Typen eine minimale aber immer noch bemerkenswerte Gewebespeicherung zumindest über gewisse Zeiträume festgestellt werden kann.

Als weiterer Nachteil ist festzustellen, dass HES nicht die ideale globuläre Struktur des Albumins aufweist und deshalb seine Grenzviskosität bedeutend höher ist als die von Albumin. Eine niedrigere Viskosität ist bei einem Plasmaexpander deshalb wünschenswert, weil nach dessen Applikation in die Zirkulation die Gesamtblutviskosität im Sinne einer Erniedrigung beeinflusst werden würde.

Es bestand daher die Aufgabe, neue verbesserte Plasmaexpander auf Amylopektinbasis zu entwickeln, die die Nachteile der fehlenden vollständigen Metabolisierbarkeit des Amylopektinderivates Hydroxyethylstärke nicht aufweisen. Gleichzeitig sollte der neue Plasmaexpander eine mehr globuläre Struktur aufweisen und damit relativ niedrigviskose Lösungen bilden.

Es kann auch als Aufgabe der Erfindung angesehen werden, weitere Einsatzgebiete für bestimmte Amylopektine zu erschließen.

Diese Aufgaben sowie weitere nicht einzeln aufgeführte Aufgaben, die sich jedoch zwanglos aus der einleitenden Erörterung ableiten lassen, werden durch den Gegenstand des Anspruchs 1 gelöst. Bevorzugte Ausgestaltungen der Erfindung sind Gegenstand der auf Anspruch 1 rückbezogenen Ansprüche.

Dadurch, dass man hyperverzweigtes Amylopektin, das einen mittleren Verzweigungsgrad, ausgedrückt als mol-% der Anhydroglucosen, die Verzweigungspunkte tragen, von zwischen > 10 und 25 mol% und ein Molekulargewicht Mw im Bereich von 40.000 bis 800.000 Dalton aufweist, und ggf. seine Derivate in Verfahren zur chirurgischen oder therapeutischen Behandlung des menschlichen oder tierischen Körpers (also von Säugern) oder in Diagnostizierverfahren einsetzt, gelingt es auf nicht ohne weiteres vorhersehbare Weise zum einen für hyperverzweigtes Amylopektin eine Reihe von neuen und interessanten Anwendungen im medizinischen Bereich zu erschließen. Zum anderen wird speziell bezogen auf den Sektor "Plasmavolumenexpansion" ein nahezu idealer, weit weniger zu gefährlichen Nebenwirkungen führender, Ersatzstoff für die zur Zeit in der Praxis noch gängigen HES-Produkte auf Stärkebasis bereit gestellt.

Im Bezug auf die Plasmavolumenexpansion wurde nämlich im Rahmen der Erfindung durch aufwendige Studien und Untersuchungen festgestellt, dass die Restfraktionen von Hydroxyethylstärke im Blutstrom und im Urin einige Stunden oder sogar Tage nach Applikation eines Plasmaexpanders eine starke Zunahme des Verzweigungsgrades aufwiesen im Vergleich zur original infundierten Hydroxyethylstärke (HES-Produkt). So stiegen die Verzweigungsgrade, ausgedrückt als mol-% der Anhydroglucosen, die Verzweigungspunkte tragen, von ca. 5 mol-% auf über 7 mol-% 2 Stunden nach Applikation und auf 8 mol-% 7 Stunden nach Applikation an. Gleichzeitig zeigte sich 48 bzw. 42 Stunden nach Infusion in den Urin-Sammelfraktionen ein noch höherer Verzweigungsgrad von 9 bzw. 10 mol-%. Dieses Phänomen wurde beobachtet unabhängig von Molekulargewicht, Substitutionsgrad oder Substitutionsmuster der applizierten Hydroxyethylstärke. Das bedeutet, dass diese Fraktionen sich beim Abbau immer mehr einer Glycogen-ähnlichen Struktur bzw. Verzweigung nähern, die in der Literatur mit ca. bis zu 10 mol-% Verzweigung angegeben wird.

Überraschenderweise wurde nun gefunden, dass die relative Stabilität der α-(1,6)-Verzweigung in Amylopektin und in Derivaten davon ausgenutzt werden kann, um den Abbau von Amylopektin gegenüber dem dominierenden α-Amylase-Abbau soweit zu reduzieren, dass ein vollständig abbaubares Polysaccharid hergestellt werden kann, welches aber immer noch die Eigenschaften eines idealen Plasmaexpanders im Bezug auf Pharmakokinetik bzw. Volumeneffekt aufweist.

Die Erfindung umfasst daher die Verwendung von hyperverzweigten Amylopektinen und von Derivaten solcher hyperverzweigten Amylopektine auf dem medizinischen Sektor.

Unter Amylopektinen versteht man dabei zunächst ganz allgemein verzweigte Stärken oder Stärkeprodukte mit α-(1-4)- und α-(1-6)-Bindungen zwischen den Glucosemolekülen. Die Verzweigungen der Kette erfolgen dabei über die α-(1-6)-Bindungen. Diese sind bei natürlich vorkommenden Amylopektinen etwa alle 15-30 Glucosesegmente unregelmäßig vorhanden. Das Molekulargewicht von natürlichem Amylopektin liegt sehr hoch im Bereich von 10⁷ bis zu 2x10⁸ Dalton. Man geht davon aus, dass auch Amylopektin in gewissen Grenzen Helices bildet.

Man kann für Amylopektine einen Verzweigungsgrad definieren. Das Maß für die Verzweigung ist das Verhältnis der Zahl von Molekülen Anhydroglucose, die Verzweigungspunkte (α-(1-6)-Bindungen) tragen, zur Gesamtzahl Moleküle der Anhydroglucose des Amylopektins, wobei dieses Verhältnis in mol-% ausgedrückt wird. In der Natur auftretendes Amylopektin weist Verzweigungsgrade von ca. 4 mol-%. Allerdings ist bekannt, dass Cluster und Molekülabschnitte von Amylopektin bei isolierter Betrachtung einen geringfügig höheren Verzweigungsgrad aufweisen als naturgemäß der Durchschnittsverzweigungsgrad.

Hyperverzweigte Amylopektine sind im Sinne der Erfindung nun solche Amylopektine, die einen über den aus der Natur für Amylopektine bekannten Verzweigungsgrad signifikant hinausgehenden Verzweigungsgrad aufweisen., Dabei handelt es sich beim Verzweigungsgrad in jedem Falle um einen Mittelwert (mittleren Verzweigungsgrad), da Amylopektine polydisperse Substanzen sind.

Solche hyperverzweigte Amylopektine weisen signifikant höhere Verzweigungsgrade, ausgedrückt als mol-% der Verzweigungsanhydroglucosen, auf im Vergleich zu unverändertem Amylopektin bzw. Hydroxyethylstärke und sind demzufolge in ihrer Struktur dem Glycogen ähnlicher.

Der für den erfindungsgemäßen Einsatz erforderlich mittlere Verzweigungsgrad der hyperverzweigten Amylopektine liegt im Bereich zwischen > 10 und 25 mol%. Dies bedeutet, dass die im Sinne der Erfindung nützlichen Amylopektine im Mittel etwa alle 10 bis 4 Glucoseeinheiten eine α-(1-6)-Bindung und damit einen Verzweigungspunkt aufweisen. Liegt der Verzweigungsgrad unterhalb von 10 mol-% ist der Abbau des verzweigten Amylopektins (z. B. beim Einsatz als Plasmaexpander) nicht ausreichend verzögert. Ist der Verzweigungsgrad größer als 25 mol-% ist der Abbau zu stark verzögert, so dass ein Einsatz beispielsweise als Plasmavolumenexpander ausscheidet.

Eine bevorzugt im medizinischen Bereich einsetzbare Amylopektintype kennzeichnet sich durch einen Verzweigungsgrad zwischen 11 und 16 mol-%.

Weitere bevorzugte hyperverzweigte Amylopektine besitzen einen Verzweigungsgrad im Bereich zwischen 13 und 16 mol-%.

Daneben kommt auch dem Molekulargewicht Mw des hyperverzweigten Amylopektins eine Bedeutung zu. Das Molekulargewicht Mw bezeichnet das Gewichtsmittel des Molekulargewichts, wie es mit einschlägigen Methoden, die diesen Mittelwert liefern, gemessen werden kann. Hierzu gehören beispielsweise wässrige GPC, HPLC, Lichtstreuung und dergleichen.

Die in der Erfindung einsetzbaren hyperverzweigten Amylopektine besitzen im Allgemeinen einen Wert für das Gewichtsmittel des Molekulargewichts Mw im Bereich von 40.000 bis 800.000 Dalton. Der untere Grenzwert für den Molekulargewichtsbereich Mw ergibt sich bei den bevorzugten Anwendungen im Wesentlichen aus der sogenannten "Nierenschwelle", die bei hyperverzweigten Verbindungen bei eben etwa 40.000 anzusetzen ist. Ist das Mw kleiner als 40.000 Dalton, würden die Moleküle zu schnell über die Niere abfiltriert werden. Oberhalb eines Mw von 800.000 Dalton wird kein zusätzlicher nennenswerter Nutzen erzielt, obwohl bei globulären Strukturen die Grenzviskosität nicht mehr vom Molekulargewicht abhängt.

Bevorzugt für den Einsatz als Plasmavolumenexpander sind Mittelwerte Mw zwischen 90.000 und 300.000 Dalton, ganz besonders zweckmäßig sind Molekulargewichte Mw zwischen 120.000 und 250.000 Dalton.

Eine besondere Ausgestaltung der Erfindung umfasst hyperverzweigtes Amylopektin, wobei der mittlere Verzweigungsgrad zwischen 11 und 16 mol% und das Molekulargewicht Mw zwischen 90.000 und 300.000 Dalton ist. Weiterhin zweckmäßige Ausgestaltungen der Erfindung schließen hyperverzweigtes Amylopektin ein, wobei der mittlere Verzweigungsgrad zwischen 13 und 16 mol% und das Molekulargewicht Mw zwischen 120.000 und 250.000 Dalton ist.

Die vorgenannten Parameter Verzweigungsgrad und Molekulargewicht gestatten eine Ziel gerichtete Beeinflussung und somit Einstellung einer gewünschten Pharmakokinetik, insbesondere das Erreichen eines erwünschten α-Amylase-Abbaus. Dem Verzweigungsgrad des Amylopektins kommt hierbei eine Schlüsselbedeutung zu. Aber auch das Molekulargewicht hat einen Einfluss auf die angesprochene Kinetik. Daneben kann es auch durch Variation der Verteilung der Verzweigungspunkte gelingen, die Kinetik des Abbaus des Amylopektins in eine gewünschte Richtung zu beeinflussen.

Von ganz besonderer Bedeutung für den Abbau des Amylopektins durch α-Amylase und damit für die Funktion als Plasmavolumenexpander ist jedoch der Verzweigungsgrad. Aufgrund des hohen Verzweigungsgrades erfolgt der Angriff der α-Amylase stark verzögert bzw. in Bereichen des Moleküls mit einer starken Dichte an Verzweigungspunkten gar nicht mehr, da dort der Zutritt der α-Amylase nicht mehr möglich ist. Solche Verbindungen sind dennoch abbaubar durch andere Enzyme bis herab zu Oligosacchariden und schließlich Glucose.

Im Bedarfsfalle können die erfindungsgemäß anzuwendenden hyperverzweigten Amylopektine derivatisert werden. Derlei Derivate umfassen chemische Abkömmlinge des Amylopektins, wie sie beispielsweise durch chemische oder biotechnologische Umsetzungen erhältlich sind.

Bevorzugte Derivate des hyperverzweigten Amylopektins sind Hydroxyethyl-, Hydroxypropyl- und Acetyl-Amylopektin. Hiervon wiederum ist Hydroxyethyl-Amylopektin ganz besonders günstig einsetzbar. Auch durch die Derivatisierung ist mithin die Kinetik des Abbaus des Amylopektins beeinflussbar. Es ist jedoch von Vorteil, dass der Grad der Derivatisierung, beispielsweise der Hydroxyethylierungsgrad, in diesen Fällen erheblich niedriger sein muss, um einen vergleichbaren Volumeneffekt bzw. eine ähnliche Pharmakokinetik aufzuweisen, im Vergleich zu einer Hydroxy-ethylstärke (HES), die aus normal verzweigtem Amylopektin hergestellt worden ist.

Die Herstellung von hyperverzweigtem Amylopektin, welches im Sinne der Erfindung unter anderem und bevorzugt zum Einsatz als Plasmaexpander geeignet ist, erfolgt in an sich bekannter Weise durch enzymatische Umwandlung durch sogenannte Verzweigungsenzyme, die die Hydrolyse der α-1,4- glycosidischen Bindungen und ihre Transformation in α-1,6-glycosidische Verbindungen katalysieren. Solche sogenannten Transfer-Enzyme können in an sich bekannter Weise z. B. aus Algen extrahiert werden gemäß PCT WO 0018893. Es sind aber auch aus dem US-Patent 4454161 und EP 0418 945 andere Glycogen-Verzweigungsenzyme bekannt, die ebenfalls entsprechend eingesetzt werden können. Die Durchführung der enzymatischen Transglycosilierung erfolgt in an sich bekannter Weise beispielsweise durch Inkubation von Wachsmaisstärke mit den entsprechenden Enzymen unter schonenden Bedingungen bei pH-Werten um ca. 7,5 und Temperaturen bei ca. 30 °C in wässriger Lösung. Die Aufarbeitung des Reaktionsansatzes erfolgt anschließend in ebenfalls bekannter Weise, wobei zuvor durch pH-Wert Veränderung bzw. Filtrationsschritte die Enzyme deaktiviert oder entfernt werden.
In einem anschließenden Hydrolyseschritt, der vorzugsweise durch Salzsäure erfolgt, wird dann das gewünschte Molekulargewicht des Produktes eingestellt. Anschließend wird das Produkt durch Diafiltration mit Membranen mit einem cut off von ca. 3.000 Dalton von niedermolekularen Verbindungen sowie Kochsalz, welches bei der Neutralisation des sauren Hydrolyseansatzes entsteht, befreit. Das Produkt wird beispielsweise durch Sprühtrocknung isoliert.

Neben dem Einsatz als Plasmavolumenexpander sind die hyperverzweigten Amylopektine auch in anderen Bereichen der Medizin nutzbringend einsetzbar.

So kann das hyperverzweigte Amylopektin bei all denjenigen Anwendungen in der Therapie und Chirurgie zum Einsatz kommen, wo auch übliche HES-Produkte auf Basis normal verzweigter Stärken einsetzbar sind.

Neben der Anwendung als Plasmavolumenexpander handelt sich hierbei vorzugsweise um den Einsatz zur Verbesserung der Mikrozirkulation, die Anwendung als Sedimentationshilfe bei der Zellseparation im Rahmen der Leukapherese oder den Einsatz zur Kryokonservierung von Blutkomponenten wie Erythrozyten oder Granulozyten.

### Modell-Beispiel 1

### Vergleichende Abbauversuche mit unterschiedlich verzweigten α-1-4 / α-1-6- Glucosacchariden

Glycogen von der Auster der Fa. SIGMA wurde durch thermoresistente α-Amylase BAN 480 L der Fa. NOVOZYMES in einer DMSO / Wassermischung mit 30 %igem Anteil an DMSO bei 70 °C und pH Wert von 6,0 abgebaut.
Der Reaktionsablauf wurde dabei durch Messung der Molekulargewichtsveränderung mittels Gelchromatographie verfolgt und nach ca. 2 Stunden wurde die Reaktion abgestoppt durch Zugabe von Natronlauge zur Enzyminaktivierung. Nach Neutralisation wurde das Produkt fraktioniert durch Ultrafiltration mittels Cellulose-Acetat-Ultrafilter mit einem nominellen cut-off von 1.000 D und 25.000 D zur Entfernung niedermolekularer Anteile sowie noch hochmolekularer Anteile. Das Produkt wurde anschließend mit Ionenaustauscher Amberlite IR 200 C sowie Aktivkohle behandelt, mit Ethanol gefällt und bei 80 °C getrocknet.

Der Verzweigungsgrad bestimmt über ¹H NMR Spektroskopie (Integration der Signale der anomeren Protonen) ergab einen Verzweigungsgrad von 15 mol%, das mittlere Molekulargewicht Mw betrug 7.000 Dalton.

Dünnkochende Wachsmaisstärke (≥ 95 % Amylopektin) (Fa. Cerestar) wurde in der gleichen Weise behandelt wie vorbeschrieben. Die isolierte, hochverzweigte Fraktion der Verzweigungscluster wies einen Verzweigungsgrad von 11 mol% auf, das mittlere Molekulargewicht Mw betrug 8.000 Dalton.

Die hochverzweigten Clusterfraktionen aus Amylopektin und Glycogen wurden danach einem Abbauversuch durch Schweinepankreas α-Amylase (Fa. Roche) in Phosphatpuffer pH 7,2 in 1%iger Lösung bei 37° C und 0,5 IU/ml Enzym unterworfen und die Abbaukinetik verfolgt durch Messung der Molekulargewichtsveränderungen mittels Gel-Chromatographie. Ebenfalls wurde ein Vergleichsversuch des Abbaus mit einem handelsüblichen Hydroxyethylstärke-Plasmaexpander durchgeführt (Voluven, Fa. Fresenius Kabi). Dabei waren deutliche Unterschiede in den Abbaukinetiken zu verzeichnen. Die Halbwertszeit des Molekulargewichts (Abbau des mittleren Molekulargewichts Mw der Ausgangssubstanz auf die Hälfte des Ausgangswertes) betrug im Falle der Fraktion mit einem Verzweigungsgrad von 15 % 60 Minuten und erreichte dabei die unter gleichen Versuchbedingungen ermittelte Halbwertszeit wie der Plasmaexpander Voluven.

Die Halbwertszeit für die Fraktion mit einem mittleren Verzweigungsgrad von 11 mol% betrug hingegen nur 25 Minuten und war damit wesentlich kürzer.

### Modell-Beispiel 2

Dünnkochende Wachsmaisstärke der Fa. Cerestar mit einem mittleren, durch NMR bestimmten Verzweigungsgrad von 4 mol% wurde entsprechend den Angaben aus Beispiel 1 einem Abbauversuch durch Schweinepankreas α-Amylase unterworfen. Hierzu wurde eine 1%ige Lösung im Phosphatpuffer pH 7,2 durch kurzes Erhitzen auf ca. 90 °C verkleistert und dem Ansatz nach Abkühlung das Enzym in einer Menge zugesetzt, dass 0,5 I.E. pro ml resultierten.
Die Versuchstemperatur betrug 37° C.

Die Abbaukinetik wurde verfolgt durch die Erfassung der Molekulargewichtsveränderungen durch Gel-Chromatographie. Unter gleichen Bedingungen wie im Beispiel 1 reduzierte sich das Molekulargewicht der Ausgangssubstanz auf den halben Wert innerhalb von 10 Minuten.
Im Vergleich zu den hochverzweigten α-1-4 / α-1-6 Glucosacchariden aus Beispiel 1 wird somit die im mittel relativ niedrigverzweigte, dünnkochende Wachsmaisstärke so schnell durch α-Amylase abgebaut, dass sie als Plasmaexpander nicht verwendbar wäre.

Damit demonstrieren die beiden Modell-Beipiele 1 und 2, dass, auch wenn die Molekulargewichte niedrig sind, eine höhere Verzweigung zu einer Verzögerung des α-Amylase-Abbaus führt und dass dieser Effekt zur Herstellung eines Plasmaexpanders einsetzbar ist.

## Patentansprüche

1. Hyperverzweigtes Amylopektin, das einen mittleren Verzweigungsgrad, ausgedrückt als mol-% der Anhydroglucosen, die Verzweigungspunkte tragen, von zwischen > 10 und 25 mol% und ein mittleres Gewichtsmittel des Molekulargewichts Mw im Bereich von 40.000 bis 800.000 Dalton aufweist, und Derivate davon zur Anwendung in Verfahren zur chirurgischen oder therapeutischen Behandlung des menschlichen oder tierischen Körpers oder in Diagnostizierverfahren.

2. Hyperverzweigtes Amylopektin nach Anspruch 1 als Plasmavolumenexpander.

3. Hyperverzweigtes Amylopektin nach Anspruch 1 zur Verbesserung der Mikrozirkulation.

4. Hyperverzweigtes Amylopektin nach Anspruch 1 als Sedimentationshilfe bei der Zellseparation im Rahmen der Leukapherese.

5. Hyperverzweigtes Amylopektin nach Anspruch 1 zur Kryokonservierung von Blutkomponenten wie Erythrozyten oder Granulozyten.

6. Hyperverzweigtes Amylopektin nach einem der vorhergehenden Ansprüche, wobei der mittlere Verzweigungsgrad zwischen 11 und 16 mol% und das Molekulargewicht Mw zwischen 90.000 und 300.000 Dalton ist.

7. Hyperverzweigtes Amylopektin nach einem der vorhergehenden Ansprüche 1 bis 5 wobei der mittlere Verzweigungsgrad zwischen 13 und 16 mol% und das Molekulargewicht Mw zwischen 120.000 und 250.000 Dalton ist.

## Claims

1. A hyperbranched amylopectin which exhibits an average degree of branching, expressed as mole % of anhydroglucoses which carry branching points, of between > 10 and 25 mole % and have a mean weight average of the molecular weight Mw in the region of 40,000 to 800,000 Dalton, and derivatives thereof for use in methods for the surgical or therapeutic treatment of the human or animal body or in diagnostic procedures.

2. The hyperbranched amylopectin according to claim 1 as a plasma volume expander.

3. The hyperbranched amylopectin according to claim 1 for improving microcirculation.

4. The hyperbranched amylopectin according to claim 1 as a sedimentation aid in cell separation in connection with leucapheresis.

5. The hyperbranched amylopectin according to claim 1 for cryo-conservation of blood components such as erythrocytes or granulocytes.

6. The hyperbranched amylopectin according to any one of the preceding claims, wherein the average degree of branching is between 11 and 16 mole % and the molecular weight Mw between 90,000 and 300,000 Dalton.

7. The hyperbranched amylopectin according to any one of preceding claims 1 to 5, wherein the average degree of branching is between 13 and 16 mole % and the molecular weight Mw between 120,000 and 250,000 Dalton.

## Revendications

1. Amylopectine hyper-ramifiée qui présente un degré de ramification moyen, exprimé en % en moles de motifs anhydroglucose porteurs de points de ramification, compris entre > 10 et 25 % en moles et une masse moléculaire moyenne en poids Mw comprise dans la plage de 40 000 à 800 000 daltons, et dérivés de celle-ci pour l'utilisation dans des procédés pour le traitement chirurgical ou thérapeutique du corps humain ou animal ou dans des procédés de diagnostic.

2. Amylopectine hyper-ramifiée selon la revendication 1 en tant que succédané de plasma.

3. Amylopectine hyper-ramifiée selon la revendication 1 pour l'amélioration de la microcirculation.

4. Amylopectine hyper-ramifiée selon la revendication 1 en tant qu'auxiliaire de sédimentation dans la séparation cellulaire dans le cadre de la leucaphérèse.

5. Amylopectine hyper-ramifiée selon la revendication 1 pour la cryoconservation de composants sanguins, tels que des érythrocytes ou granulocytes.

6. Amylopectine hyper-ramifiée selon l'une des revendications précédentes, dans laquelle le degré de ramification moyen se situe entre 11 et 16 % en moles et le poids moléculaire Mw entre 90 000 et 300 000 daltons.

7. Amylopectine hyper-ramifiée selon l'une des revendications précédentes 1 à 5, dans laquelle le degré de ramification moyen se situe entre 13 et 16 % en moles et le poids moléculaire Mw entre 120 000 et 250 000 daltons.
